# EUROPEAN PATENT SPECIFICATION

(11) **EP 3 787 418 B1**
(45) Date of publication and mention of the grant of the patent: **29.05.2024**
(21) Application number: 19726518.4
(22) Date of filing: 18.04.2019
(51) Int. Cl.: A23L 33/135, A23L 33/18, C07K 14/47, A23L 33/00

(54) **FORMULA WITH SPECIFIC BETA-LACTOGLOBULIN PEPTIDES**
FORMEL MIT SPEZIFISCHEN BETA-LACTOGLOBULIN-PEPTIDEN
FORMULE AVEC DES PEPTIDES DE BÊTA-LACTOGLOBULINE SPÉCIFIQUES

(30) Priority: 30.04.2018 WO PCT/NL2018/050281
(43) Date of publication of application: 10.03.2021
(73) Proprietor: N.V. Nutricia, 2712 HM Zoetermeer (NL)
(72) Inventor: GOUW, Joost Willem, 3584 CT Utrecht (NL); NEULENBROEK, Laura Antoinette Petronella Maria, 3584 CT Utrecht (NL); JO, Juandy, 427048 Singapore (SG); KNIPPELS, Leon Matthieu Johannes, 3584 CT Utrecht (NL); GARSSEN, Johan, 3584 CT Utrecht (NL)
(74) Representative: Nederlandsch Octrooibureau
(86) International application number: PCT/NL2019/050236
(87) International publication number: WO 2019/212331

(56) References cited:
- WO-A1-2016/148572
- WO-A1-2017/144730
- WO-A2-2009/082229

## Description

### FIELD

The invention is in the field of formulae comprising hydrolysed cow's milk protein for infants that suffer from or are at risk of developing cow's milk protein allergy.

### BACKGROUND

Dietary proteins are exposed to the immune system in the gastro-intestinal tract. As a default response of the immune system oral tolerance is developed to these innocuous food proteins. The development of oral immune tolerance is highly present in infants, who get exposed to numerous harmless proteins in early life by exposure to dietary proteins. A derail of this natural response to harmless proteins takes place, food allergy will occur. It is generally accepted that the prevalence of food allergy has been increasing in recent decades, particularly in westernised countries. In early life, cow's milk allergy is the most dominant food allergy with currently 2-5% of infants suffer from cow's milk allergy and a higher percentage is at risk of developing the allergy.

For infants suffering from allergy to cow's milk protein, infant formulae are on the market comprising extensively hydrolysed proteins (extensive protein hydrolysate) or even solely free amino acids as nitrogen source. In these formulae hardly or no allergenic protein or peptides are present.

Infants born from parents of whom one or both suffers from an atopic disease, or who have one or more siblings suffering from an atopic disease, have a higher risk of becoming allergic to dietary proteins. For this group, besides the preferred breast feeding, hypoallergenic formulae are available on the market, comprising a partial protein hydrolysate (partially hydrolysed proteins). These partially hydrolysed proteins have a decreased allergenicity. This approach has been demonstrated to be efficient in order to prevent sensitization by native proteins present in the adapted formulae. Typically, the extent of hydrolysis of proteins is less than those of extensively hydrolysed proteins for infants already suffering from allergy. These formulations have the advantage of not only reducing the risk of developing an allergic response by preventing sensitization to the protein, but also support the natural development of oral immune tolerance to the intact protein. This brings the advantage that later on the native protein can be introduced in the diet, with a reduced risk on allergic reactions.

The strict avoidance of allergens for the prevention and management of allergen sensitivity has been advised for many decades. A focus on complete food allergen avoidance may inadvertently have contributed to an increase in food allergy in the recent decades. A better way to prevent or treat allergy is to develop strategies that promote the oral tolerance induction of allergens, such as oral immune therapy or presenting food allergens in a form that favour the induction of natural mechanisms of tolerance (Allen et al, 2009, Pediatr Allerg Immunol 20:415-422).

EP 2 044 851 discloses a nutritional composition with partially hydrolysed milk protein having a degree of hydrolysis between 15 % and 25 % and 50 to 1000 ng of TGF-beta per 100 ml for the primary prevention of allergic reactions to dietary protein and the prevention of development of atopic diseases in young mammals.

EP 0 629 350 discloses the use of non-allergenic whey protein hydrolysates which are said to be capable of inducing cow's milk protein tolerance.

EP 0 827 697 discloses the use of whey, that has been hydrolysed enzymatically for the preparation of compositions that induce oral tolerance to cows' milk in susceptible mammals. The whey has a level of immunological detection of allergenic proteins >= 100 times less than that of unhydrolysed whey.

WO 00/42863 discloses a hypoallergenic composition for the induction of protein tolerance in at risk infants of protein allergy, comprising a non-allergenic protein extensively hydrolysed basis and/or a free amino acid basis, said composition comprising as the active ingredient at least one tolerogenic peptide of the allergenic protein.

WO 2011/151059 discloses infant nutrition with partially hydrolysed proteins and non-digestible oligosaccharides for use in induction of oral tolerance against native dietary proteins.

WO 2015/090347 relates to identified peptides of that are capable of inducing tolerance to cow's milk, especially to β-lactoglobulin.

WO 2017/144730 discloses a strategy for achieving desensitisation or induction of tolerance to milk protein allergens, e.g. β-lactoglobulin, in humans or animals, comprising formulating and using a composition comprising a purified intact expressed milk protein together with one or more purified peptides from said intact milk protein.

WO 2016/0148572 discloses oral immune tolerance with a specific mixture of synthetic betalactoglobulin peptides and probiotics.

However, the hypoallergenic compositions in the prior art often provide their effects just by avoiding the presence of potential allergens - thereby not solving the underlying problem - and/or by requiring the presence of special ingredients such as growth factors or synthetic or purified peptides. Furthermore the peptides identified in the prior art may not be suitable to induce oral immune tolerance in a wide part of the human population. So still there exists a need for a nutrition comprising a specific hydrolysate of bovine beta-lactoglobulin with specific peptides and components that further enhance the oral immune tolerance for subjects at risk of developing or suffering from food allergy with improved effects on oral immune tolerance induction against food proteins, in particular cow's milk protein.

Thus, the technical problem underlying the present invention is to provide compositions, methods, uses and means for overcoming the above-identified disadvantages, in particular for providing an improved induction of oral immune tolerance against dietary proteins in humans, in particular humans at risk for developing a food allergy, in particular infants, and in particular infants at risk for developing cow's milk protein allergy.

### SUMMARY OF THE INVENTION

The inventors contribute to the belief in the art that a better way to prevent or treat allergy is to develop strategies that promote the oral tolerance induction of allergens, such as oral immune therapy or presenting food allergens in a form that favour the induction of natural mechanisms of tolerance (Allen et al, 2009, Pediatr Allerg Immunol 20:415-422).

The foundation for all T-cell responses, including the induction of regulatory T cells (Tregs), consists of three interconnected processes; T-cell receptor activation, co-stimulation and cytokine signalling. Activation of the T-cell receptor on CD4⁺ T cells is facilitated by the recognition of specific peptides, called T-cell epitopes, presented by MHC class II molecules on antigen-presenting cells. The presence of T cell epitopes in a protein hydrolysate is essential for the induction of cow's milk-specific Tregs. Amino acid (AA) #13-48 of mature betalactoglobulin (BLG) is of particular interest because synthetic peptides covering this region are able, in a preventive setting, to significantly reduce the acute allergic skin response in a murine model for cow's milk allergy. Taken together, this region in BLG appears to be important for tolerance development towards whey protein.

The investigators found - using a new method - that a specific partially hydrolysed whey-based infant formula contained specific, exact beta-lactoglobulin (BLG) peptides that function as T-cell epitopes to support the development of oral tolerance to cow's milk protein across a wider population of human subjects, for example having different HLA-DRB1 alleles. These specific alleles are the most relevant, as HLA-DR alleles are the predominant isotype of MHC class II and play a central role in CD4+ T-cell selection and activation. HLA-DR is a heterodimer molecule comprised of HLA-DRA and HLA-DRB chains. While the HLA-DRA gene is highly conserved in humans, there are multiple HLA-DRB genes. Among those, HLA-DRB1 is the most polymorphic gene and it is expressed five times higher than its functional paralogs,

Firstly, a novel liquid chromatography-mass spectrometry (LC-MS) method was developed to identify a limited list of specific, exact BLG-derived peptides that were naturally present with the highest incidence in a formula with whey protein hydrolysate, with a focus on region AA#13-48 of the mature bovine BLG. The inventors could identify in total thirteen BLG-peptides of minimal nine amino acids in this area of interest, six of which were identified consistently over different production batches.

Secondly, the formula was subjected to the Prolmmune ProPresent^{®} antigen presentation assay and a MHC class II binding algorithm to identify the relevant HLA-DRB1-restricted peptides. It was confirmed that these peptides were processed and presented by human dendritic cells. The above peptides were clustered in two unique sequence groups, i.e. DIQ... DIS (AA#11-30) and AMA... APL (AA#23-39). It was assessed that fragments of the identified two unique sequence groups of BLG had high affinity to bind to the selected HLA-DRB1 alleles. It was found that a mixture of BLG peptides was most preferred to ensure coverage of interaction with several HLA-DRB1 alleles commonly present in the human population. Particularly a mixture of peptides comprising at least BLG peptide SEQ ID NO: 5 together with one of BLG peptides SEQ ID NO: 2, 3 or 4 covers most HLA-DRB1 alleles tested. Reference is made to examples 2 and 3.

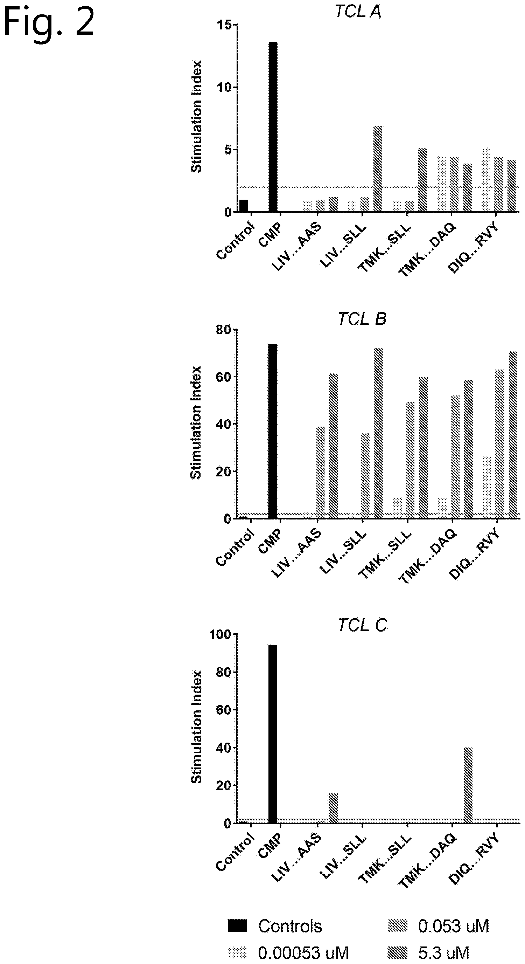

Thirdly, the sequences of the identified BLG peptides were tested and found to be recognized by human cow's milk-specific T-cell lines obtained from different allergic donors and induce T-cell proliferation/activation. Reference is made to example 4 and figure 2 associated therewith. When tested on these different T-cell lines different patterns of T cell reactivity were observed, and while not every peptide reacted with each cell line, when taken together (in particular BLG peptide SEQ ID NO: 4 alone, or a combination of BLG peptide SEQ ID NO: 1 with one of SEQ ID NOs: 2, 3 or 5) reactivity of every cell line was covered. These results are indicative for an improved effect of hydrolysates with these exact BLG-derived peptides on the development of oral immune tolerance to whey protein, or a decreased risk of developing oral immune tolerance.

As evidenced in example 6, the peptides have a higher number of MHC Class II binding domains and thus an increased likelihood to be presented to T cells.

The BLG peptides in the region AA#13-48 provide a further improved effect on oral immune tolerance induction or a further decreased risk of developing oral immune intolerance when consumed together with a strain of a lactic acid producing bacterium all or not together with non-digestible oligosaccharides, and therefore these results are also indicative for further improved effects of compositions with hydrolysed protein comprising a mixture of such BLG-derived peptides consisting of an amino acid sequence corresponding to the consecutive amino acids of the beta-lactoglobulin protein represented by SEQ ID NO: 1, SEQ ID NO: 2, SEQ ID NO: 3, SEQ ID NO: 4 and SEQ ID NO: 5, preferably together with a strain of a lactic acid producing bacterium, all or not together with non-digestible oligosaccharides (probiotics or synbiotics).

Furthermore, in the attached experimental part it is shown that the presence of lactic acid producing bacteria was found to increase the expression of HLA-DR molecules on the surface of the dendritic cells (example 6). An increased expression of HLA-DR is also indicative for an improved presentation of peptides to T cells, and hence oral immune tolerance induction.

### DETAILED DESCRIPTION OF THE INVENTION

The present invention thus concerns a nutritional composition comprising a protein hydrolysate from mammalian milk,
- for use in induction of oral immune tolerance against milk protein and/or
- for use in prevention or treatment of oral immune intolerance against milk protein and/or
- for reducing the risk of developing oral immune intolerance against milk protein and/or
in a human subject, wherein the composition comprises at least a peptide having an amino acid sequence according to SEQ ID NO: 5 and at least one peptide having a sequence according to SEQ ID NO: 2, SEQ ID NO: 3 or SEQ ID NO: 4, In a preferred embodiment, the composition comprises even all five peptides selected from this group.

These peptides are herein also referred to as "BLG-derived peptides". The composition preferably further comprises a strain of a lactic acid-producing bacterium, more preferably further comprises one or more non-digestible oligosaccharides (NDO(s)) as defined further below. The mammalian milk is preferably milk from a species of the genus *Bos, Bison, Bubalus* or *Capra,* more preferably from genus *Bos*, preferably from cow's milk. Also, the invention pertains to
a nutritional composition according to claim 1 for use in prevention or treatment of oral immune intolerance against milk protein and/or for reducing the risk of developing oral immune intolerance against milk protein in a human subject, preferably against milk protein from a species of the genus *Bos, Bison, Bubalus* or *Capra,* more preferably from genus *Bos*, preferably against cow's milk, , said composition preferably further comprising a strain of a lactic acid-producing bacterium, more preferably comprising one or more NDO(s) as defined further below.

In one embodiment,
- induction of oral immune tolerance against milk protein and/or
- prevention or treatment of oral immune intolerance against milk protein and/or
- reduction of the risk of developing oral immune intolerance against milk protein concerns milk protein from a particular mammalian species and the hydrolysed milk protein comprised in the composition is from the same mammalian species, preferably both are from the genus *Bos*.

Associated therewith, the invention also pertains to a nutritional composition comprising:
a. a strain of lactic acid-producing bacterium belonging to the genus *Bifidobacterium;*
b. a milk protein hydrolysate from a species of the genus *Bos, Bison, Bubalus or Capra,* more preferably from genus *Bos*, wherein the milk protein hydrolysate comprises at least a peptide having a sequence according to SEQ ID NO: 5 and at least one peptide having a sequence according to SEQ ID NO: 2, SEQ ID NO: 3 or SEQ ID NO: 4
c. less than 6 µg allergenic beta-lactoglobulin, preferably less than 3.5 µg beta-lactoglobulin, per g of total protein,
d. less than 10 wt% of peptides or proteins having a size of 5 kDa or above, based on total protein,
e. at least 50 wt%, preferably at least 95 wt% of hydrolysed whey protein based on total protein,
f. optionally one or more non-digestible oligosaccharide(s) selected from the group consisting of fructooligosaccharide, non-digestible dextrin, galactooligosaccharide, xylooligosaccharide, arabino-oligosaccharide, arabinogalacto-oligosaccharide, gluco-oligosaccharide, glucomanno-oligosaccharide, galactomanno-oligosaccharide, mannan-oligosaccharide, chito-oligosaccharide, uronic acid oligosaccharide, sialyloligosaccharide, and fucooligosaccharide, and mixtures thereof, preferably fructo-oligosaccharides, and
g. optionally long-chain polyunsaturated fatty acids, preferably docosahexaenoic acid (DHA), more preferably at least 0.35 wt.% DHA based on total fatty acids.

The nutritional composition in the context of the present invention is defined here below, and all embodiments apply to all aspects of the invention, including the composition and the composition for use according to the invention.

### Peptides/hydrolysate

The composition according to the invention comprises at least a peptide having a sequence according to SEQ ID NO: 5 and at least one peptide having a sequence according to SEQ ID NO: 2, SEQ ID NO: 3 or SEQ ID NO: 4. These peptides are derived or derivable from beta-lactoglobulin and thus addressed as beta-lactoglobulin-derived peptides. Throughout the specification and claims, the amino acid sequences of the BLG-peptides with SEQ ID NO: 1 - 5 are identified here below:
SEQ ID NO: 1: XIVTQTMKGLDIQKVAGTWYSLAMAAS
SEQ ID NO: 2: XIVTQTMKGLDIQKVAGTWYSLAMAASDISLL
SEQ ID NO: 3: TMKGLDIQKVAGTWYSLAMAASDISLL
SEQ ID NO: 4: TMKGLDIQKVAGTWYSLAMAASDISLLDAQ
SEQ ID NO: 5: DIQKVAGTWYSLAMAASDISLLDAQSAPLRVY

Herein, X defines an amino acid selected from leucine (L) or isoleucine (I), preferably X is leucine. SEQ ID NO: 1 wherein X = L is herein also referred to as SEQ ID NO: 9. SEQ ID NO: 1 wherein X = I is herein also referred to as SEQ ID NO: 37. SEQ ID NO: 2 wherein X = L is herein also referred to as SEQ ID NO: 11. SEQ ID NO: 2 wherein X = I is herein also referred to as SEQ ID NO: 38. The choice for X being either leucine or isoleucine is a direct consequence of the source of mammalian milk. Leucine is preferred, since mammalian milk from the genus *Bos*, preferably cow's milk, is most preferred.

The claimed mixture of peptides is based on the Prolmmune ProPresent^{®} antigen presentation assay where this mixture of peptides was found to cover more HLA-DRB1 alleles tested.

The peptides are provided by a protein hydrolysate (i.e. hydrolysed proteins) derived from mammalian milk, preferably milk from a species of the genus *Bos, Bison, Bubalus* or *Capra,* more preferably from genus *Bos*, most preferably from cow's milk (*Bos taurus*)*.* The amino acid sequence of BLG in species of *Bos* and *Bison* are similar, and SEQ ID NOs: 9, 11, 3, 4 and 5 are obtainable in hydrolysates from milk protein of these species. In species of *Capra* and *Bubalus* amino acid 1 is different (an isoleucine instead of a leucine in SEQ ID NOs: 37 and 38), and SEQ ID NOs: 37, 38, 3, 4 and 5 are obtainable in hydrolysates from milk protein of these species. In a preferred embodiment, the peptides are derived from whey protein. The nutritional composition preferably comprises at least 50 wt%, more preferably at least 70 wt%, even more preferably at least 95 wt% of hydrolysed whey protein based on total protein. A suitable source is a mixture of acid whey protein and demineralised sweet whey protein. Acid whey and sweet whey are commercially available. Sweet whey is the by-product of rennet-coagulated cheese and comprises caseinoglycomacropeptide (CGMP), and acid whey (also called sour whey) is the by-product of acid-coagulated cheese, and does not contain CGMP. Suitable sources for the whey protein are demineralised whey (Deminal, Friesland Campina, the Netherlands) and/or whey protein concentrate (WPC80, Friesland Campina, the Netherlands). The whey protein preferably comprises acid whey, more preferably at least 50 wt%, more preferably at least 70 wt% acid whey, based on total whey protein. Acid whey has an improved amino acid profile compared to sweet whey protein.

Hydrolysis may be achieved using a mixture of microbial endopeptidases and exopeptidases using the method as described in example 1 of WO2011/151059. Hydrolysates with the BLG-peptides according to SEQ ID NO: 1, SEQ ID NO: 2, SEQ ID NO: 3, SEQ ID NO: 4 and SEQ ID NO: 5 can preferably be prepared using the recipe according to example 1. Preferably a mixture of an endoprotease and exoprotease is employed. To determine whether any given composition comprises one or more of the BLG-derived peptides, means for identification are described in the experimental parts.

The composition preferably comprises less than 10 wt%, preferably less than 6 wt% of peptides or proteins with a size of 5 kDa or above, based on total protein. In one embodiment, the composition comprises more than 0.3 wt%, preferably more than 0.5 wt%, more preferably more than 1 wt%, even more preferably more than 1.5 wt% of these peptides with a size of 5 kDa or above, based on total protein. It is preferred that more than 1 wt% of peptides or proteins present in the composition has a size of 1 kDa or above, based on total protein, more preferably at least 5 wt%, more preferably at least 10 wt%, based on total protein. It is more preferred that more than 1 wt% of peptides or proteins present in the composition has a size of 3 kDa or above, based on total protein, more preferably at least 5 wt%, more preferably at least 10 wt%, based on total protein.

The size distribution of the peptides in the protein hydrolysate can be determined by means of size exclusion high pressure liquid chromatography as known in the art. Saint-Sauveur et al. "Immunomodulating properties of a whey protein isolate, its enzymatic digest and peptide fractions" Int. Dairy Journal (2008) vol. 18(3) pages 260-270 describes an example thereof. In short, the total surface area of the chromatograms is integrated and separated into mass ranges expressed as percentage of the total surface area. The mass ranges are calibrated using peptides/proteins with a known molecular mass. The protein hydrolysate comprising said peptides is preferably characterised in that it comprises between 64 and 89 wt% peptides with a molecular weight below 1000 Da, between 10 and 30 wt% peptides with a molecular weight between 1000 and 5000 Da and between 1 and 6 wt% of peptides or proteins with a molecular weight above 5 kDa, all based on total protein.

While the peptide weight distribution is informative, it does not provide any information about the sequence and activity of the peptides present in the formula. Structural information is crucial to understand the overall biological activity a hydrolysed infant formula might possess.

Consistently several specific peptides that originated from a region in BLG known to contain T-cell epitopes have been identified in different production batches. Only two studies investigated the peptide profile of pHP previously (Wada et al. Peptides 2015 73:101-105; Catala-Clariana et al, Electrophoresis 2013 Jul;34(13):1886-1894). Although these studies focused on bioactive peptides in general and not specifically on T-cell epitopes, none of the peptides with SEQ ID NO: 1 - 5 identified in the present application were found in these previously tested products. In addition to the profiles of these hydrolyzed protein products, the peptide profile of a non-commercial available BLG-based hydrolysate has been determined (Pecquet et al J Allergy Clin Immunol 2000 Mar;105(3):514-521). Also in this hydrolysate, two peptides (AA#21-40 and AA#25 -40) that overlap with the region of interest in suit, but again no peptides with SEQ ID NO: 1- 5 were identified. In at least two of the three studies reported above, a hydrolysate from a different manufacturer was investigated (Wada, and Pecquets) and as indicated above the main reason for these differences might be differences in manufacturing processes. This is further corroborated by a recent study in which extensively hydrolysed infant formulas from different manufactures were characterized with peptidomics. The authors showed that infant formulas from different manufacturers had a distinct signature based on their peptide profile and therefore might have a different effect in clinical trials (Lambers et al. Food Sci Nutr 2015 Jan;3(1):81-90. Hochwallner et al, 2017, Allergy 72: 416-425).

In a preferred embodiment of the present invention, the present composition according to claim 11 comprises, per gram of total protein, at least 10 mcg (microgram), more preferably 10 to 5000 mcg, more preferably 20 to 2000 mcg, more suitable 30 to 500 mcg and particularly preferably 50 to 250 mcg of the sum of the beta-lactoglobulin-derived peptide(s) consisting of an amino acid sequence represented by SEQ ID NO: 1, SEQ ID NO: 2,
SEQ ID NO: 3, SEQ ID NO: 4 and SEQ ID NO: 5. The BLG-derived peptides are preferably present in therapeutically effective amounts.

It is preferred that the nutritional composition comprises less than 6 µg allergenic beta-lactoglobulin BLG per g protein, preferably less than 5 µg per g protein, more preferably less than 3.5 µg per g protein. In the context of the invention, the expression 'allergenic beta-lactoglobulin' refers to intact or immunogenic BLG, and does not account for the hydrolysed BLG. It is preferred that the nutritional composition comprises an amount of allergenic beta-lactoglobulin above 0.8 µg per g protein. Allergenic BLG is determined by ELISA as known in the art and an amount exceeding 0.8 µg per g protein is indicative for a partial protein hydrolysate. For sake of comparison, an extensively hydrolysed whey protein exhibits less than 0.2 µg allergenic BLG per g protein. Small but significant amounts of allergenic BLG together with the hydrolysed BLG or BLG-derived peptides, more preferably also in combination with a strain of lactic-acid producing bacterium, even more preferably also in combination with a lactic-acid producing bacterium and one or more NDO(s), enhances the tolerogenic capacity of the composition, yet the amount of BLG is still sufficiently low to reduce the allergenicity of the nutritional composition and for the nutritional composition to be hypoallergenic. From this perspective it is preferred to use a protein hydrolysate comprising the minor amount of allergenic beta-lactoglobulin and comprising the BLG-derived peptides according to the invention, over synthetic peptides consisting of an amino acid sequence represented by SEQ ID NO: 1, SEQ ID NO: 2, SEQ ID NO: 3, SEQ ID NO: 4 and SEQ ID NO: 5.

### Lactic acid producing bacteria

The composition according to the invention comprises a strain of lactic acid producing bacterium species, which enhances the oral immune tolerogenic capacity of the BLG peptides of the invention. The bacterium strain is a probiotic belonging to the genus *Bifidobacterium.* Evidence is provided in examples 5 and 7. The presence of lactic acid producing bacteria was found to increase the expression of HLA-DR molecules on the surface of the dendritic cells. An increased expression of HLA-DR is also indicative for an improved presentation of peptides to T cells, and hence oral immune tolerance induction.

Suitable lactic acid producing bacteria are strains of the genus *Bifidobacteria* (e.g. *B. breve, B. longum, B. infantis, B.* bifidum).

*Bifidobacterium breve* and *Bifidobacterium longum* are especially suitable lactic acid producing bacteria.

The composition comprises a strain of lactic acid-producing bacterium belonging to the genus *Bifidobacterium,* preferably to the species *Bifidobacterium breve.* The *B*. *breve* preferably has at least 95 % identity of the 16 S rRNA sequence when compared to the type strain of *B*. *breve* ATCC 15700, more preferably at least 97% identity (Stackebrandt & Goebel, 1994, Int. J. Syst. Bacteriol. 44:846-849). Suitable *B. breve* strains may be isolated from the faeces of healthy human milk-fed infants. Typically, these are commercially available from producers of lactic acid bacteria, but they can also be directly isolated from faeces, identified, characterised and produced. According to one embodiment, the present composition contains a *B*. *breve* selected from the group consisting of *B*. *breve* Bb-03 (Rhodia/Danisco), *B. breve* M-16V (Morinaga), *B. breve* R0070 (Institute Rosell, Lallemand), *B. breve* BR03 (Probiotical), *B. breve* BR92) (Cell Biotech), DSM 20091, LMG 11613, YIT4065, FERM BP-6223 and CNCM 1-2219.

*B. breve* can be *B. breve* M-16V and *B. breve* CNCM 1-2219, most preferably *B*. *breve* M-16V. *B*. *breve* I-2219 was published in WO 2004/093899 and was deposited at the Collection Nationale de Cultures de Microorganisms, Institute Pasteur, Paris, France on 31 May 1999 by Compagnie Gervais Danone. *B. breve* M-16V was deposited as BCCM/LMG23729 and is commercially available from Morinaga Milk Industry Co., Ltd.

The lactic acid producing bacterium may be present in the composition at any suitable concentration, preferably in a therapeutically effective amount or "amount effective for treating" in the context of the invention. Preferably, the lactic acid producing bacterium strain is included in the present composition in an amount of 10⁴ - 10¹³ cfu per g dry weight of the composition, preferably 10⁵ - 10¹¹ cfu/g, most preferably 10⁶ - 10¹⁰ cfu/g.

### Non-digestible oligosaccharides

In a preferred embodiment, the present composition additionally comprises one or more non-digestible oligosaccharides [NDO]. Like the lactic acid-producing bacteria, these further increase the oral immune tolerance inducing properties of peptides of the BLG protein. Evidence of an enhanced effect is given in example 5.

Advantageously and most preferred, the non-digestible oligosaccharide is water-soluble (according to the method disclosed in L. Prosky et al, J. Assoc. Anal. Chem 71: 1017-1023, 1988) and is preferably an oligosaccharide with a degree of polymerisation (DP) of 2 to 200. The average DP of the non-digestible oligosaccharide is preferably below 200, more preferably below 100, even more preferably below 60, most preferably below 40.

The non-digestible oligosaccharide is preferably a prebiotic. It is not digested in the intestine by the action of digestive enzymes present in the human upper digestive tract (small intestine and stomach). The non-digestible oligosaccharide is fermented by the human intestinal microbiota. For example, glucose, fructose, galactose, sucrose, lactose, maltose and the maltodextrins are considered digestible. The oligosaccharide raw materials may comprise monosaccharides such as glucose, fructose, fucose, galactose, rhamnose, xylose, glucuronic acid, GalNac etc., but these are not part of the oligosaccharides. The non-digestible oligosaccharide is preferably selected from the group consisting of fructooligosaccharide, non-digestible dextrin, galactooligosaccharide, xylooligosaccharide, arabino-oligosaccharide, arabinogalacto-oligosaccharide, gluco-oligosaccharide, glucomanno-oligosaccharide, galactomanno-oligosaccharide, mannan-oligosaccharide, chito-oligosaccharide, uronic acid oligosaccharide, sialyloligosaccharide and fucooligosaccharide, and mixtures thereof, preferably fructo-oligosaccharides. Examples of sialyloligosaccharide are 3-sialyllactose, 6" sialyllactose, sialyllacto-N-tetraoses, disialyllactoNtertraoses. Examples of fucooligosaccharides are (un)sulphated fucoidan oligosaccharides, 2'fucosyllactose, 3' fucosyllactose, lacto-N-fucopentaose I, II, III, LNDH, lactodifucotetraose, lacto-N difucohexaose I and II.

One suitable type of oligosaccharide is a short-chain oligosaccharide which has an average degree of polymerisation of less than 10, preferably at most 8, preferably in the range of 2 - 7. The short-chain oligosaccharide preferably comprises galacto-oligosaccharides and/or fructo-oligosaccharides (i.e. scGOS and/or scFOS). In one embodiment, the composition comprises galacto-oligosaccharides, preferablybeta-galacto-oligosaccharides, preferably trans-galacto-oligosaccharides. The galacto-oligosaccharides preferably have an average degree of polymerisation in the range of 2 - 8, preferably 3 - 7, i.e. are short-chain oligosaccharides in the context of the invention. (Trans)galactooligosaccharides are for example available under the trade name Vivinal^{®}GOS (Friesland Campina Domo Ingredients, Netherlands), Bimuno (Clasado), Cup-oligo (Nissin Sugar) and Oligomate55 (Yakult). The composition preferably comprises short-chain fructo-oligosaccharides and/or short-chain galacto-oligosaccharides, preferably at least short-chain fructo-oligosaccharides. Fructooligosaccharides may be inulin hydrolysate products having an average DP within the aforementioned (sub-) ranges; such FOS products are for instance commercially available as Raftilose P95 (Orafti) or with Cosucra.

Another suitable type of oligosaccharide is long-chain fructo-oligosaccharides (IcFOS) which has an average degree of polymerisation above 10, typically in the range of 10 - 100, preferably 15 - 50, most preferably above 20. A particular type of long-chain fructo-oligosaccharides is inulin, such as Raftilin HP.

The present composition may contain a mixture of two or more types of non-digestible oligosaccharides, most preferably a mixture of two non-digestible oligosaccharides. In case the NDO comprises or consists of a mixture of two distinct oligosaccharides, one oligosaccharide may be short-chain as defined above and one oligosaccharide may be long-chain as defined above. Most preferably, short-chain oligosaccharides and long-chain oligosaccharides are present in a weight ratio short-chain to long-chain in the range of 1:99 - 99:1, more preferably 1:1 - 99:1, more preferably 4:1 - 97:3, even more preferably 5:1 - 95:5, even more preferably 7:1 - 95:5, even more preferably 8:1 - 10:1, most preferably about 9:1.

In one embodiment, the composition comprises at least two of fructo-oligosaccharides and/or galacto-oligosaccharides. Suitable mixtures include mixtures of long-chain fructo-oligosaccharides with short-chain fructo-oligosaccharides or with short-chain galacto-oligosaccharides, most preferably long-chain fructo-oligosaccharides with short-chain fructo-oligosaccharides.

The present composition preferably comprises 0.05 to 20 wt% of said non-digestible oligosaccharides, more preferably 0.5 to 15 wt%, even more preferably 1 to 10 wt%, most preferably 2 to 10 wt%, based on dry weight of the present composition. When in liquid form, the present composition preferably comprises 0.01 to 2.5 wt% non-digestible oligosaccharide, more preferably 0.05 to 1.5 wt%, even more preferably 0.25 to 1.5 wt%, most preferably 0.5 - 1.25 wt%, based on 100 ml.

In one embodiment, the NDO mixture does not comprises any detectable amounts of acid oligosaccharides.

When the non-digestible oligosaccharide is a mixture, the averages of the respective parameters are used for defining the present invention.

The combination of a NDO and a lactic acid producing bacterium as defined here above is also referred to as a "synbiotic". The presence of therapeutically effective amounts of the NDO together with the lactic acid-producing bacterium are believed to further improve the oral immune tolerance inducing properties A strain of *Bifidobacterium,* preferably *B*. *breve,* together with fructo-oligosaccharides. Reference is made to example 5.

### Other components

The composition may further comprise long chain polyunsaturated fatty acids (LC-PUFA). LC-PUFA are fatty acids wherein the acyl chain has a length of 20 to 24 carbon atoms (preferably 20 or 22 carbon atoms) and wherein the acyl chain comprises at least two unsaturated bonds between said carbon atoms in the acyl chain. More preferably the present composition comprises at least one LC-PUFA selected from the group consisting of eicosapentaenoic acid (EPA, 20:5 n3), docosahexaenoic acid (DHA, 22:6 n3), arachidonic acid (ARA, 20:4 n6) and docosapentaenoic acid (DPA, 22:5 n3), preferably DHA, EPA and/or ARA. Such LC-PUFAs have a further beneficial effect on reducing the risk for allergy. The preferred content of LC-PUFA in the present composition does not exceed 15 wt.% of total fatty acids, preferably does not exceed 10 wt.%, even more preferably does not exceed 5 wt.%. Preferably the present composition comprises at least 0.2 wt.%, preferably at least 0.25 wt.%, more preferably at least 0.35 wt.%, even more preferably at least 0.5 wt.% LC-PUFA of total fatty acids, more preferably DHA. The present composition preferably comprises ARA and DHA, wherein the weight ratio ARA/DHA preferably is above 0.25, preferably above 0.5, more preferably 0.75 - 2, even more preferably 0.75-1.25. The weight ratio is preferably below 20, more preferably between 0.5 and 5. The amount of DHA is preferably above 0.2 wt%, more preferably above 0.3 wt%, more preferably at least 0.35 wt%, even more preferably 0.35 - 0.6 wt% on total fatty acids.

### Nutritional composition

The composition according to the invention is used as a nutritional composition, nutritional therapy, nutritional support, as a medical food, as a food for special medical purposes or as a nutritional supplement. The present composition is an enteral (oral) composition. The composition is administered orally to, or intended to be administered orally to, a subject in need thereof, in particular to children and infants, including toddlers, preferably children up to 6 years of age, preferably infants or young children typically with an age of 0 - 36 months, more preferably infants 0-12 months of age, most preferably 0 - 6 months of age (use not covered by the present invention).

Thus, in some embodiments, the present composition is an infant formula, follow-on formula or young child formula (also referred to as growing-up milk), preferably it is an infant formula or follow-on formula, most preferably an infant formula. The term 'infant formula' is well-defined and controlled internationally and consistently by regulatory bodies. In particular, CODEX STAN 73 - 1981 "Standard For Infant Formula and Formulas For Special Medical Purposes Intended for Infants*"* is widely accepted. It recommends for nutritional value and formula composition, which require the prepared milk to contain per 100 ml not less than 60 kcal (250 kJ) and no more than 70 kcal (295 kJ) of energy. FDA and other regulatory bodies have set nutrient requirements in accordance therewith.

Preferably, the present enteral, nutritional composition is for providing the daily nutritional requirements to a human, in particular for administration to, in particular for feeding, humans, in particular infants including toddlers, preferably at risk for developing milk protein allergy, preferably against milk protein from a species of the genus *Bos, Bison, Bubalus* or *Capra,* more preferably from genus *Bos*, most preferably cow's milk protein allergy. In one embodiment, the milk protein allergy concerns milk from a particular mammalian species and the hydrolysed milk protein comprised in the composition is form the same mammalian species, preferably both are from the genus *Bos*.

In order to meet the caloric requirements of the infant, the present enteral composition preferably comprises 50 to 200 kcal/100 ml liquid, more preferably 60 to 90 kcal/100 ml liquid, even more preferably 60 to 75 kcal/100 ml liquid. This caloric density ensures an optimal ratio between water and calorie consumption. The osmolarity of the present composition is preferably between 150 and 420 mOsmol/l, more preferably 260 to 320 mOsmol/l. The low osmolarity aims to reduce the gastrointestinal stress.

Preferably, the present enteral composition is in a liquid form, preferably with a viscosity below 35 mPa.s, more preferably below 6 mPa.s as measured in a Brookfield viscometer at 20°C at a shear rate of 100 s-1. Suitably, the present enteral composition is in a powdered from, which preferably can be reconstituted with water to form a liquid, or in a liquid concentrate form, which should be diluted with water. When the present enteral composition is in a liquid form, the preferred volume administered on a daily basis is in the range of about 80 to 2500 ml, more preferably about 450 to 1000 ml per day.

The composition according to the invention preferably comprises a lipid component, preferably a lipid component suitable for infant nutrition as known in the art. The lipid component of the present composition preferably provides 2.9 to 6.0 g, more preferably 4 to 6 g per 100 kcal of the composition. When in liquid form, the composition preferably comprises 2.1 to 6.5 g lipid per 100 ml, more preferably 3.0 to 4.0 g per 100 ml. Based on dry weight the present infant or follow on formula preferably comprises 12.5 to 40 wt% lipid, more preferably 19 to 30 wt%.

The composition according to the invention may comprise further proteinaceous material, in addition to the beta-lactoglobulin-derived peptide(s) according to the invention. In the context of the present invention the additional "protein" or "proteinaceous material" or "protein equivalents" encompasses proteins, peptides, free amino acids and partially or extensively hydrolysed proteins.

Preferably, the further proteinaceous material - additional to the beta-lactoglobulin-derived peptide(s) - does not evoke an allergic reaction or is hypoallergenic, such as free amino acids, and hydrolysed protein. As a further protein component, i.e. apart from the beta-lactoglobulin-derived peptide(s), the composition according to the present invention preferably comprises free amino acids, hydrolysed whey protein, preferably partially hydrolysed whey proteins.

The composition according to the present invention preferably contains less than 1 wt% intact mammalian (cow)'s milk protein. The composition may comprise an additional protein component selected from the group consisting of free amino acids, hydrolysed whey protein and proteins from other sources such as soy, pea, rice, collagen or the like, in intact form, in partially hydrolysed form, and/or in extensively hydrolysed form.

The present composition preferably contains at least 50 wt% protein component derived from non-human milk, more preferably at least 90 wt%, based on dry weight of total protein.

The present composition preferably contains 4 to 25 %, more preferably 5 to 20 %, more preferably 7 to 16 %, most preferably 7 to 12 % protein, based on total calories. The present composition, when in liquid form, preferably contains 0.5 to 6.0 g, more preferably 0.8 to 3.0 g, even more preferably 1.0 to 2.5 g of protein per 100 ml. The present composition preferably comprises at least 7.0 wt%, more preferably at least 8.0 wt%, most preferably at least 9 or at least 10 wt% protein based on dry weight of the total composition. Preferably, the present composition comprises at most 40 wt%, more preferably at most 15 wt%, preferably at most 20 wt% of protein based on dry weight of the total composition.

The composition may comprise digestible carbohydrate(s). Typically, digestible carbohydrates that are known in the art to be suitable for use in infant nutritional compositions are used, for example selected from digestible polysaccharides (e.g. starch, maltodextrin), digestible monosaccharides (e.g. glucose, fructose), and digestible disaccharides (e.g. lactose, sucrose). Particularly suitable is lactose and/or maltodextrin. In one embodiment, the composition does not comprise lactose.

The digestible carbohydrate component preferably comprises at least 60 wt% lactose based on total digestible carbohydrate, more preferably at least 75 wt%, even more preferably at least 90 wt% lactose based on total digestible carbohydrate.

### Human subjects

The human subjects or population targeted is preferably infants (0-12 months), preferably infants at risk of developing allergy, preferably milk protein allergy, preferably milk protein from a species of the genus *Bos, Bison, Bubalus* or *Capra,* more preferably from genus *Bos*, most preferably cow's milk protein allergy, preferably infants suffering from milk protein allergy preferably against milk protein from a species of the genus *Bos, Bison, Bubalus* or *Capra,* more preferably from genus *Bos*, most preferably cow's milk protein allergy. Infants at risk are preferably determined by the family history, preferably at least one parent being or having a history of being allergic.

The human being preferably has a specific HLA-DRB1 allele selected form the groups consisting of HLA-DRB1 *01:01, HLA-DRB1*04:01, HLA-DRB1*04:04, HLA-DRB1*04:05, HLA-DRB1 7:01 and HLA-DRB1*09:01. The HLA-DRB1 is the most predominant human MHC class II isotypes (>90%) the HLA-DRB1 gene locus is polymorphic while HLA-DRA1 gene locus is monomorphic (i.e., HLA-DRB1 genotype determines the whole HLA-DR molecule), and HLA-DRB1 allele is expressed five times higher than its paralogs (HLA-DRB3, -DRB4 or-DRB5) and is present in all human beings.

### Oral immune tolerance induction

The nutritional composition according to claim 1 is for use in
- the induction of oral immune tolerance against milk protein preferably against milk protein from a species of the genus *Bos, Bison, Bubalus* or *Capra,* more preferably from genus *Bos*, most preferably cow's milk protein in a human subject or population, or
- prevention or treatment of oral immune intolerance against milk protein preferably against milk protein from a species of the genus *Bos, Bison, Bubalus or Capra,* more preferably from genus *Bos*, most preferably cow's milk protein in a human subject or population, or
- reducing the risk of development of oral immune intolerance against milk protein preferably against milk protein from a species of the genus *Bos, Bison, Bubalus or Capra,* more preferably from genus *Bos*, most preferably cows' milk protein, in a human subject or population,

As addressed here above, the human subject or population is preferably an infant subject or population. In the context of the present invention the term 'oral tolerance' means oral immune tolerance.

In a preferred embodiment, the invention pertains to a nutritional composition as defined here above, for use in prevention of oral immune intolerance against cow's milk protein in an infant subject or infant population at risk of developing milk protein allergy, preferably against milk protein from a species of the genus *Bos, Bison, Bubalus* or *Capra,* more preferably from genus *Bos*, most preferably cow's milk protein allergy.

### FIGURE LEGENDS

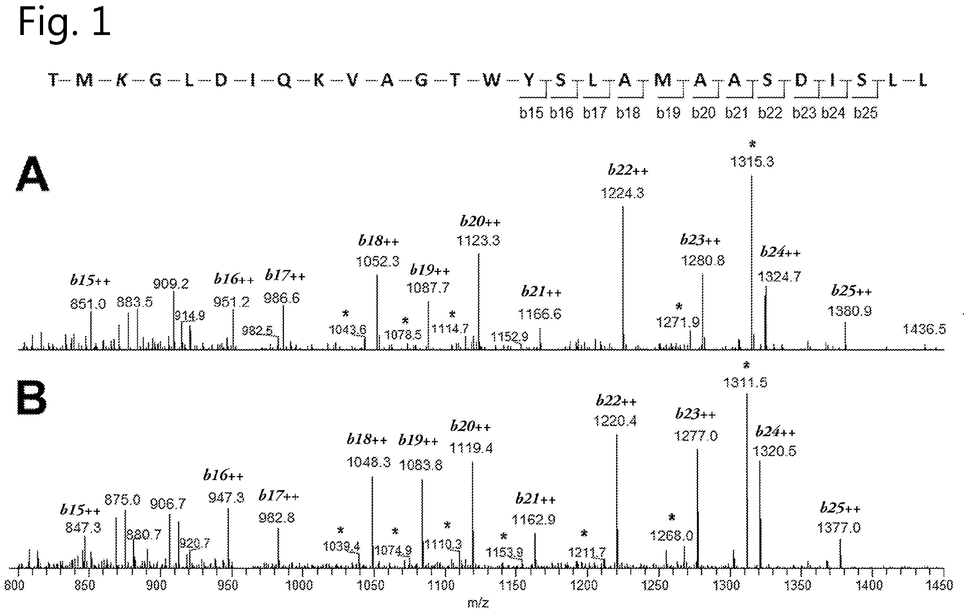

**Figure 1****:** Comparison of fragmentation spectra of a representative peptide (SEQ ID NO: 3; shown at the top) experimentally recorded (B) and its synthetic equivalent (A). The mass difference between the annotated fragment ions of the experimental and the synthetic peptide corresponds to the presence of a stable isotope labelled lysine in the synthetic peptide, italic in the sequence (+8 Da). The * indicates water loss fragments of the corresponding b-ions.
**Figure 2****:** T cell responses after stimulation with the identified peptides. Cow's milk-specific T cell lines (TCLs) of three different donors were stimulated with synthetic equivalents of the identified peptides (LIV...AAS = SEQ ID NO: 9; LIV...SLL = SEQ ID NO: 11; TMK...SLL = SEQ ID NO: 3; TMK...DAQ = SEQ ID NO: 4; DIQ...RVY = SEQ ID NO: 5). Cow's milk protein (CMP) was taken along as control. A stimulation index ≥ 2 was considered significant.

### Example 1: Hydrolysed whey protein and new method for identification of betalactoglobulin peptides

A mixture of acid whey protein and demineralised sweet whey protein (wt ratio protein 77:23) was dissolved in water (purified by reversed osmosis) and afterwards hydrolysed under specific conditions. Acid whey and sweet whey are commercially available. Sweet whey is the by-product of rennet-coagulated cheese and comprises caseinoglycomacropeptide (CGMP), and acid whey (also called sour whey) is the by-product of acid-coagulated cheese, and does not contain CGMP. Suitable sources for the acid whey protein are demineralised whey (Deminal, Friesland Campina, the Netherlands) and whey protein concentrate (WPC80, Friesland Campina, the Netherlands). A mixture of microbial endopeptidases and exopeptidases was used for hydrolysing these two protein sources using the method as described in example 1 of WO 2011/151059. Subsequently, the hydrolysed protein solution was spray dried. The size distribution of the peptides in this protein hydrolysate was determined by means of size exclusion high pressure liquid chromatography as known in the art. In short the total surface area of the chromatograms is integrated and separated into mass ranges expressed as percentage of the total surface area. The mass ranges are calibrated using peptides/proteins with a known molecular mass. The whey protein hydrolysate can be categorized as a partial or moderate protein hydrolysate.

The resulting hydrolysate powder was used as sole protein source in infant formulas. 10 different batches of infant fomulae were produced in a similar way. The amount of betalactoglobulin as determined by ELISA method as known in the art was between about 0.8 and 3.5 µg/g total protein.

In order to determine the presence of specific sequences in biological samples, mass spectrometry (MS) is the method of choice as opposed to the more traditional techniques that are currently used to characterize protein hydrolysates. A recent development in MS, termed peptidomics, allows for the characterization of peptide sequences with great sensitivity and specificity (Dallas et al. J Nutr 2015;145:425-433). This technique is capable of closing the gap between understanding the impact of sequence specificity in relation to the biological activity a protein hydrolysate might have.

Samples were essentially prepared as described by Butré et al with the addition of a reduction and alkylation step (Butre et al. Anal Bioanal Chem 2014;406):5827-5841). All chemicals were obtained from Sigma Aldrich. Briefly, the partial Hydrolysate protein (pHP) batches were diluted to 0.5% (v/v) using 50 mM ammonium bicarbonate followed by the reduction of peptides with 4 mM DTT and alkylation with 8 mM iodoacetamide. The mixture was cleared by centrifugation at 20,000 × g for 10 min and diluted to 0.1% (v/v) using 0.1 M acetic acid.

All samples were analysed by nanoflow liquid chromatography using an Agilent 1200 HPLC system (Agilent Technologies) coupled on-line to a LTQ Velos mass spectrometer (Thermo Fisher Scientific).

The liquid chromatography part of the system was operated in a setup essentially as described previously (14). Peptides were trapped at 5 µl/min in 100% solvent A (0.1 M acetic acid in water) on a 2-cm trap column (100-µm inner diameter, packed in-house using Aqua C18, 5-µm resin (Phenomenex)) and eluted to a 20-cm IntegraFrit column (50-µm inner diameter, ReproSil-Pur C18-AQ 3-µm, New Objective) at ~100 nl/min in a 90-min gradient from 10 to 40% solvent B (0.1 M acetic acid in 8:2 (v/v) acetonitrile/water). The eluent was sprayed via standard coated emitter tips (New Objective) butt-connected to the analytical column. The mass spectrometer was operated in data-dependent mode, automatically switching between MS and MS/MS. Full scan mass spectra (from m/z 300 to 1,200) were acquired at zoom scan rate after accumulation to a target value of 3,000. The five most intense ions at a threshold above 500 were selected for collision-induced at normalized collision energy of 35% after accumulation to a target value of 10,000.

All MS data was processed by Proteome Discoverer (version 2.1, Thermo Scientific). Peak lists were generated using a standard workflow. Peptide identification was performed by searching individual peak lists of CID fragmentation spectra against a database containing selected bovine whey and casein proteins using Mascot (version 2.4.1, Matrix Science). No enzyme was specified and no missed cleavages were allowed. Precursor ion mass tolerance was set to 0.2 Da and product ion mass tolerance to 0.5 Da. Carbamidomethylation (C) was set as fixed modification.

Of the 314 peptides identified by this approach, 101 could be assigned to betalactoglobulin (BLG) leading to a sequence coverage of BLG of 90%. Most of the remaining peptides originated from other abundant whey proteins such as α-lactalbumin and serum albumin. In total 13 betalactoglobulin peptides of minimal 9 AAs were identified in the region of interest (AA#13-48) (See Table 1 and Figure 1). Six of them were identified consistently in all 10 batches. Unambiguous peptide identifications were obtained by comparing characteristics (retention time, peptide mass and fragmentation spectrum) of the experimental peptide with its stable-isotope labelled synthetic equivalent. An example is shown in Figure 1 where the fragmentation spectra of the experimental and synthetic peptide show excellent correlation. In this way the identity of the six peptides found in all 10 batches was confirmed.

**Table 1. Sequences of the beta-lactoglobulin peptides identified in 10 different batched of a partially hydrolysed whey-based infant formula**

| Amino acid number in BLG sequence | Sequence | Number of batches where the peptide was identified to be present | SEQ ID NO |
|---|---|---|---|
| AA 1-22 | LIVTQTMKGLDIQKVAGTWYSL | 7 | 6 |
| AA 1-25 | LIVTQTMKGLDIQKVAGTWYSLAMA | 9 | 7 |
| AA 1-26 | LIVTQTMKGLDIQKVAGTWYSLAMAA | 10 | 8 |
| AA 1-27 | LIVTQTMKGLDIQKVAGTWYSLAMAAS | 10 | 9 |
| AA 1-30 | LIVTQTMKGLDIQKVAGTWYSLAMAASDIS | 2 | 10 |
| AA 1-32 | LIVTQTMKGLDIQKVAGTWYSLAMAASDISLL | 10 | 11 |
| AA 1-35 | LIVTQTMKGLDIQKVAGTWYSLAMAASDISLLDAQ | 5 | 12 |
| AA 1-39 | LIVTQTMKGLDIQKVAGTWYSLAMAASDISLLDAQSAPL | 2 | 13 |
| AA 1-42 | LIVTQTMKGLDIQKVAGTWYSLAMAASDISLLDAQSAPLRVY | 2 | 14 |
| AA 5-35 | QTMKGLDIQKVAGTWYSLAMAASDISLLDAQ | 2 | 15 |
| AA 6-32 | TMKGLDIQKVAGTWYSLAMAASDISLL | 10 | 3 |
| AA 6-35 | TMKGLDIQKVAGTWYSLAMAASDISLLDAQ | 10 | 4 |
| AA 11-42 | DIQKVAGTWYSLAMAASDISLLDAQSAPLRVY | 10 | 5 |

A similar analysis was performed with batches of Nutrilon pepti, an extensively hydrolysed whey protein containing infant formula marketed for the dietary management of cow's milk allergy. The BLG peptides of Table 1 were not present in Nutrilon pepti.

### Example 2 In vitro identification of HLA-DR-restricted peptides

Identification of BLG-derived peptides presented by human DCs was performed by Prolmmune, as described (Lamberth et al, Sci Transl Med 2017 11;9(372):10.1126/scitranslmed.aag1286.). Briefly, peripheral blood mononuclear cell samples from 12 HLA-DR-typed healthy adult donors were obtained. The donors were selected based on common 11 HLA-DRB1 alleles (Table 2). The HLA-DRB1 was prioritized in this study due to the facts that the HLA-DR molecule is the most predominant human MHC class II isotypes (>90%) (Sturniolo et al. Nat Biotechnol 1999;17:555-561.) and that HLA-DRB1 gene locus is polymorphic while HLA-DRA1 gene locus is monomorphic (i.e., HLA-DRB1 genotype determines the whole HLA-DR molecule) (Marsh et al, 2010. Tissue Antigens 75:291-455.). Furthermore, HLA-DRB1 allele is expressed five times higher than its paralogs (HLA-DRB3, -DRB4 or -DRB5) and is present in all individuals (O'Leary et al. Nucleic Acids Res 2016 4;44(D1):D733-45.).

**Table 2: Donors with HLA-DRB1 typing information**

| Donor ID | DRB1_1 | DRB1_2 |
|---|---|---|
| P1 | *03:01 | *03:01 |
| P2 | *01:01 | *04:01 |
| P3 | *01:01 | *07:01 |
| P4 | *01:01 | *04:05 |
| P5 | *04:01 | *07:01 |
| P6 | *13:02 | *14:01 |
| P7 | *03:01 | *09:01 |
| P8 | *11:01 | *15:01 |
| P9 | *03:01 | *11:01 |
| P10 | *07:01 | *15:01 |
| P11 | *03:01 | *15:01 |
| P12 | *01:01 | *04:04 |

Immature monocyte-derived DCs were generated in vitro and matured in the presence of tested hydrolysed protein (HP). DCs were harvested and lysed in order to obtain HLA-DR complexes by using a specific immunoaffinity method. Peptides were eluted from the HLA-DR complexes and subsequently analyzed by high resolution sequencing LC-MS/MS. The presence of six endogenous relevant proteins (i.e., ITGAM, ApoB, CLIP, TFRC, FcER2/FcGR2 and LAMP-1/3) was assessed as a control for this assay. Each donor sample had to express a minimum of 3 relevant proteins to be qualified for subsequent analysis. The resulting data of HLA-DR-restricted peptides were compiled and assessed using sequence analysis software referencing the Swiss-Prot Human Proteome Database with the incorporated test item sequences. The likelihood of peptides to be real findings is described by their expect value ≤ 0.05. The false discovery rate was determined to be < 1%.

Using the ProPresent^{®} antigen presentation assay by focusing on BLG-derived sequences, 15 relevant peptides with an expect value ≤ 0.05 were identified within 5 donor samples (Table 3). These peptides could be further grouped into 2 unique sequence groups, i.e., DIQ...DIS (AA#11-30) and AMA...APL (AA#23-39) (Table 4). Importantly, both sequence groups overlapped with the region of interest (i.e., AA#13-48 of mature BLG). This finding demonstrated that BLG-derived peptides of interest can be presented by HLA-DR molecules on human DCs when incubated with a specific HP.

**Table 3. Significant fragments identified from peptide-HLA-DR complexes with expect value < 0.05.**

| Donor ID | DRB1_1 | DRB1_2 | Sequence of Fragment (Mature BLG) [SEQ ID NO] | Amino Acid Start/End | Expect Value |
|---|---|---|---|---|---|
| P2 | *01:01 | *04:01 | AMAASDISLLDAQSAPL - [16] | 23-39 | 0.043 |
| | | | - MAASDISLLDAQSAPLR [17] | 24-40 | 0.0013 |
| P3 | *01:01 | *07:01 | - MAASDISLLDAQSAPL - [18] | 24-39 | 0.009 |
| P4 | *01:01 | *04:05 | DIQKVAGTWYSLAMAASDI - [19] | 11-29 | 0.000052 |
| | | | - - - - VAGTWYSLAMAASDIS [20] | 15-30 | 0.00021 |
| | | | ------ GTWYSLAMAASDIS [21] | 17-30 | 0.00037 |
| P7 | *03:01 | *09:01 | DIQKVAGTWYSLAMAASDI - - [19] | 11-29 | 0.00019 |
| | | | DIQKVAGTWYSLAMAASDIS - [22] | 11-30 | 0.00053 |
| | | | - - - - VAGTWYSLAMAASDI - - [23] | 15-29 | 0.002 |
| | | | - - - - VAGTWYSLAMAASDIS - [20] | 15-30 | 0.000019 |
| | | | - - -- - AGTWYSLAMAASDIS - [24] | 16-30 | 0.0014 |
| | | | - - - - - - GTWYSLAMAASDI - - [25] | 17-29 | 0.00023 |
| | | | - - - - - - GTWYSLAMAASDIS - [21] | 17-30 | 0.000015 |
| P12 | *01:01 | *04:04 | AMAASDISLLDAQSAPL - [16] | 23-39 | 0.028 |
| | | | - MAASDISLLDAQSAPL - [18] | 24-39 | 0.042 |

**Table 4. HLA-DR allele association on unique region of mature BLG with significant fragments were defined by having expect value < 0.05.**

| Donor ID | DRB1_1 | DRB1_2 | Unique Region (Mature BLG) [SEQ ID NO] | Amino acid start - end | Number of Significant Fragments and individual fragment |
|---|---|---|---|---|---|
| P2 | *01:01 | *04:01 | AMAASDISLLDAQSAPLR [39] | 23-40 | 2 (23-39 and 24-40) |
| P3 | *01:01 | *07:01 | MAASDISLLDAQSAPL [18] | 24-39 | 1 (24-39) |
| P4 | *01:01 | *04:05 | DIQKVAGTWYSLAMAASDIS [22] | 11-30 | 3 (11-29, 15-30, and 17-30) |
| | | | VAGTWYSLAMAASDIS [20] | | |
| | | | GTWYSLAMAASDIS [21] | | |
| P7 | *03:01 | *09:01 | DIQKVAGTWYSLAMAASDIS [22] | 11-30 | 7 (11-29, 11-30, 15-29, 15-30, 16-30, 17-29, 17-30) |
| P12 | *01:01 | *04:04 | AMAASDISLLDAQSAPL [16] | 23-39 | 2 (23-39, 24-39) |

Not all donor antigen presenting cells bound to peptides. This concerned HLA-DRB1*03:01 in homozygous donor P1. Similar arguments could be applied for donors P6 and P11.

### Example 3 MHC Class II Binding In Silico Assessment

A limitation of the ProPresent^{®} antigen presentation assay is the usage of general anti-HLA-DR antibody, and not a specific antibody against a particular HLA-DRB1 allele (e.g., anti-DRB1*01:01 antibody), to isolate peptide-HLA-DR complexes of interest. Hence, with a donor with heterozygous genotypes of HLA-DRB1, e.g., *01:01 and *04:01, it is uncertain which allele will present the identified peptide.

Hence, the identified HLA-DR-restricted peptides were computed into the IEDB MHC Class II Binding Prediction software (http://tools.iedb.org/mhcii/) in order to assess binding of discovered peptides to the selected HLA-DRB1 alleles, as described (Wang P, et al. PLoS Comput Biol 2008 4;4(4):e1000048.; Wang et al, BMC Bioinformatics 2010, 11:568-2105-11-568.) The default IEDB recommended prediction method was selected. For each peptide sequence (15 mers long), a percentile rank was generated by comparing the peptide's score against the scores of five million random 15 mers selected from the Swiss-Prot database. A lower percentile rank indicates a higher affinity of peptide binding to a particular MHC class II allele, in which the IEDB recommends to make a selection based on a consensus percentile rank of the top 10%. In order to be more stringent with the in silico assessment, a selection based on a consensus percentile rank of the top 3% was made.

The MHC class II prediction software was utilized to assess whether fragments of the identified two unique sequence groups of BLG would have high affinity to bind to the selected HLA-DRB1 alleles of the characterized donors from the ProPresent^{®} assay, i.e., HLA-DRB1 *01:01, *03:01, *04:01, *04:04, *04:05, *07:01 and *09:01. As shown in Table 5, with an arbitrary threshold of percentile rank set at < 3% (i.e., top 3% high binders), fragments of DIQ...DIS (AA#11 -30) were predicted to have high affinity to bind to 5 HLA-DRB1 alleles, i.e., DRB1*01:01, *04:01, *04:04, *04:05 and *09:01. In contrast, it was estimated that only one fragment of AMA...APL (AA#23 -39) bound to HLA-DRB1*07:01 with high affinity. Taken together, these findings suggest that fragments from AA#11-30 had a higher likelihood to be presented as T-cell epitopes than the ones from AA#23-39. Moreover, the data suggest that several common HLA-DRB1 alleles could present fragments derived from the identified two BLG-derived unique sequence groups.

These unique sequence groups of BLG (AA#11-30 and #23-39) overlapped and could be joined into one sequence (i.e., AA#11-39). This joined sequence was highly correlated with one constantly identified peptide in the tested HP (AA#11 -42; DIQ...RVY). Therefore the sequence of AA#11-42 was subjected into the MHC class II prediction software. Importantly, the predicted complexes of AA#11-42 reconfirmed the predicted results of two *in vitro* identified unique sequences (Table 6), suggesting that both unique sequences of BLG could be derived from the AA#11-42 peptide that exists in the tested formula. Furthermore, parts of AA#11-42 were predicted to have high affinity to the same sets of identified HLA-DRB1 alleles, i.e., DRB1*01:01, *04:01, *04:04, *04:05, *07:01 and *09:01. In conclusion, the in silico assessment confirms the *in vitro* findings that BLG-derived peptides can bind to common HLA-DRB1 alleles.

**Table 5. In silico assessment on potential peptide-HLA-DRB1 complexes of two identified BLG-derived peptides with percentile rank < 3%.**

| HLA-DRB1 Allele | Predicted 15-mer Fragment of *DIQKVAGTWYSLAMAASDIS* (SEQ ID NO: 25) or *AMAASDISLLDAQSAPL* (SEQ ID NO: 19) [SEQ ID NO] | Percentile Rank |
|---|---|---|
| HLA-DRB1 *09:01 | AGTWYSLAMAASDIS [24] | 0.86 |
| HLA-DRB1 *04:05 | AGTWYSLAMAASDIS [24] | 1.38 |
| HLA-DRB1 *04:05 | VAGTWYSLAMAASDI [23] | 1.40 |
| HLA-DRB1 *09:01 | VAGTWYSLAMAASDI [23] | 1.56 |
| HLA-DRB1 *04:05 | KVAGTWYSLAMAASD [26] | 2.26 |
| HLA-DRB1*01:01 | AGTWYSLAMAASDIS [24] | 2.37 |
| HLA-DRB1 *04:04 | AGTWYSLAMAASDIS [24] | 2.44 |
| HLA-DRB1 *09:01 | KVAGTWYSLAMAASD [26] | 2.53 |
| HLA-DRB1 *04:01 | AGTWYSLAMAASDIS [24] | 2.84 |
| HLA-DRB1*07:01 | AASDISLLDAQSAPL [27] | 2.39 |

**Table 6. In silico assessment on potential peptide-HLA-DRB1 complexes of a BLG-derived long peptide with percentile rank < 3 (top 3% binders).**

| HLA-DRB1 Allele | Predicted 15-mer Fragment of *DIQKVAGTWYSLAMAASDISLLDAQSAPLR VY* (SEQ ID NO: 5) [SEQ ID NO] | Percentile Rank |
|---|---|---|
| HLA-DRB1 *09:01 | GTWYSLAMAASDISL [28] | 0.65 |
| HLA-DRB1 *09:01 | AGTWYSLAMAASDIS [24] | 0.86 |
| HLA-DRB1 *04:05 | GTWYSLAMAASDISL [28] | 1.30 |
| HLA-DRB1 *09:01 | TWYSLAMAASDISLL [29] | 1.32 |
| HLA-DRB1 *04:05 | AGTWYSLAMAASDIS [24] | 1.38 |
| HLA-DRB1 *04:05 | VAGTWYSLAMAASDI [23] | 1.40 |
| HLA-DRB1 *04:05 | TWYSLAMAASDISLL [29] | 1.40 |
| HLA-DRB1 *09:01 | VAGTWYSLAMAASDI [23] | 1.56 |
| HLA-DRB1 *09:01 | WYSLAMAASDISLLD [30] | 2.12 |
| HLA-DRB1 *09:01 | YSLAMAASDISLLDA [31] | 2.14 |
| HLA-DRB1*01:01 | GTWYSLAMAASDISL [28] | 2.18 |
| HLA-DRB1 *04:05 | KVAGTWYSLAMAASD [26] | 2.26 |
| HLA-DRB1*01:01 | AGTWYSLAMAASDIS [24] | 2.37 |
| HLA-DRB1*07:01 | AASDISLLDAQSAPL [27] | 2.39 |
| HLA-DRB1*07:01 | SDISLLDAQSAPLRV [32] | 2.44 |
| HLA-DRB1 *04:04 | AGTWYSLAMAASDIS [24] | 2.44 |
| HLA-DRB1 *04:04 | GTWYSLAMAASDISL [28] | 2.44 |
| HLA-DRB1 *04:04 | TWYSLAMAASDISLL [29] | 2.44 |
| HLA-DRB1*01:01 | TWYSLAMAASDISLL [29] | 2.46 |
| HLA-DRB1 *09:01 | KVAGTWYSLAMAASD [26] | 2.53 |
| HLA-DRB1 *04:04 | YSLAMAASDISLLDA [31] | 2.57 |
| HLA-DRB1 *04:01 | GTWYSLAMAASDISL [28] | 2.83 |
| HLA-DRB1 *04:01 | AGTWYSLAMAASDIS [24] | 2.84 |
| HLA-DRB1 *04:04 | WYSLAMAASDISLLD [30] | 2.91 |

Several *in silico* assessments confirmed the *in vitro* ProPresent^{®} findings on the peptide-HLA-DR complexes. However, we observed that some *in silico* predicted complexes did not correlate to the ones found in the ProPresent^{®} assay, e.g., DRB1*04:04 with AGT...DIS. This discrepancy can be explained either by a small size of tested cohort in the ProPresent^{®} assay (n=12), by the fact that each donor had a different combination of HLA-DRB1 allele types (i.e., interindividual variation) and/or by a tendency of *in silico* assessment to be over predictive (thus requires a stringent cut-off). Therefore, it is prudent to combine both *in vitro* and *in silico* approaches in order to identify peptide-MHC class II complexes.

### Example 4 T-Cell Proliferation Assay

Synthetic peptides were obtained from JPT technologies. Only peptides containing at least 9 AAs were taken into account, as this is the minimal size for binding to MHC class II molecules and subsequent T-cell recognition (Holland et al, Front Immunol 2013 Jul 1;4:172.) Synthetic peptides (JPT technologies) identical to the peptides identified in all batches of the HP of example 1 were dissolved in dimethylsulfoxide (Sigma Aldrich) at a concentration of 5.3 mM. The peptides were tested on cow's milk-specific T-cell lines (TCLs) from three different donors. The peptides were tested on cow's milk protein-specific T-cell lines (TCLs) from three infant donors, diagnosed with cow's milk protein allergy, aged <1 year, 7.5 months and 6 years old. These TCLs were generated previously and have been shown to recognize epitopes in the region of interest (AA#13-48 of mature BLG). Proliferation was determined as described previously (Ruiter et al, Clin Exp Allergy 2006, 36(3):303-310). Stimulation indexes (Sls, ratio between proliferation of allergen/peptide-stimulated and non-stimulated T cells) were calculated and a SI ≥ 2 was considered positive.

To confirm that the peptides identified in HP were recognized by T cells, synthetic peptides identical to these identified peptides were tested on cow's milk-specific human TCLs. As there was a considered overlap between the identified peptides, five peptides were tested (Table 7) that were identified in all batches and differed with more than 9A from each other. All tested peptides were able to induce proliferation (Figure 2). However, each donor showed a different recognition pattern. This was also seen with the *in silico* prediction data (data not shown). All five peptides induced proliferation of TCL B suggesting that this TCL recognizes either the overlapping part of the peptides (AA#11 -27) or multiple T-cell epitopes in this region. Also TCL A showed a proliferative response after stimulation with several peptides. As peptide LIV...AAS (AA#1-27) was not able to induce proliferation in TCL A while peptide LIV...SLL (AA#1-32) did, the region containing AA#28-32 (DISLL ) was essential for the response of TCL A. Unexpectedly, TCL C recognized peptide LIV...AAS (AA#1-27), while the longer peptide containing the same sequence with 5 additional AAs, LIV...SLL (AA#1-32), was not able to induce a proliferative response indicating that longer peptides are not always presented better.

**Table 7. Sequences of the beta-lactoglobulin peptides identified in 10 different batched of a partially hydrolysed whey-based infant formula and tested for T cell proliferation activity**

| Amino acid number in BLG sequence | Sequence | SEQ ID NO |
|---|---|---|
| AA 1-27 | LIVTQTMKGLDIQKVAGTWYSLAMAAS | 9 |
| AA 1-32 | LIVTQTMKGLDIQKVAGTWYSLAMAASDISLL | 11 |
| AA 6-32 | TMKGLDIQKVAGTWYSLAMAASDISLL | 3 |
| AA 6-35 | TMKGLDIQKVAGTWYSLAMAASDISLLDAQ | 4 |
| AA 11-42 | DIQKVAGTWYSLAMAASDISLLDAQSAPLRVY | 5 |

Not all peptides were recognized by each donor, which is an indicative for the variation between donors. Each donor expressed a different panel of MHC class II molecules. To determine if and which HLA-DRB1 allele expressed by the donors presented the peptides the same algorithm as described above was used. The in silico prediction confirmed that HLA-DRB1 alleles (*11:01 & *04:04) expressed by donor B were able to present all peptides. Based on the algorithm data TCL A should only recognize peptide DIQ...RVY (AA#11-42), however, this TCL showed a proliferative response after stimulation with four of the five peptides. A possible explanation might be that the other three peptides were not presented by HLA-DRB1 but by other MHC class II molecules.

In conclusion, example 1 to 4 demonstrate that a specific whey protein hydrolysate contains functional T-cell epitopes. Specific, beta-lactoglobulin peptides were identified to be present, that are HLA-DRB1 restricted peptides. These are presented to and recognized by T cells from different donors, including healthy donors and cow's milk protein allergic infants. This interaction was confirmed by *in silico* analysis. The HP may therefore stimulate the development of oral immunotolerance to protein.

### Example 5: Synbiotic mixture shows a further improved effect of tolerance induction compared to probiotics or prebiotics alone

Several published studies support a notion that dietary antigens (including peptides) within the intestinal lumen can be absorbed and subsequently captured and presented by intestinal antigen-presenting cells, i.e., antigen sampling mechanisms, without the need of a disrupted intestinal barrier. There are at least two distinct mechanisms that continuously work to sample dietary antigens at the healthy state, i.e., microfold /M cell-mediated transcytosis and goblet-cell-associated antigen passage. Both pathways provide dietary antigens to intestinal lamina propria CD103+ DCs, which in return could imprint gut-homing molecules on T and B cells, could promote differentiation of intestinal IgA-producing plasma cells as well as could generate intestinal Tregs, a key player in the state of hyporesponsiveness to fed antigen known as oral tolerance. This suggests that peptides within the tested HP can be orally absorbed, followed by antigen capture and presentation by intestinal CD103+ DCs and subsequently culminated in generation of functional Tregs.

For the development of oral tolerance, T-cell epitopes within the tested HP need to be presented under the right circumstances. In addition to TCR activation, co-stimulation and cytokine signalling play an important role in the generation of Tregs. Pro- or prebiotics play an important role in creating the right environment for this predisposition towards Tregs. The preventive effect of synthetic BLG peptides was strengthened in combination with a probiotic or prebiotic diet.

To demonstrate the effect of pro-, pre- and synbiotics an experiment was performed similar as described in example 4 of WO 2016/148572, with the same protocol, and the same concentration and mixture of synthetic peptides (`Pepmix'), the pepmix identified in Table 8.

**Table 8. pepmix according to WO 2016/148572**

| Peptide | | Sequence |
|---|---|---|
| 1 (18AA) | SEQ ID NO: 33 | QKVAGTWYSLAMAASDIS |
| 2 (18AA) | SEQ ID NO: 34 | WYSLAMAASDISLLDAQS |
| 3 (18AA) | SEQ ID NO: 35 | AASDISLLDAQSAPLRVY |
| 4 (18AA) | SEQ ID NO: 36 | LLDAQSAPLRVYVEELKP |

A number of diets were compared:
(a) synbiotic diet of example 1 of WO 2016/14472 (1 wt% scFOS/lcFOS in a wt ratio 9:1 + 2 wt% 2×10⁹ cfu/g *Bifidobacterium breve* M-16V,
(b) probiotic diet of example 4 of WO 2016/148572 with 2 wt% 2×10⁹ cfu/g *Bifidobacterium breve* M-16V, and
(c) prebiotic diet containing 1 wt% of scFOS/lcFOS wt ratio 9/1.

The short-chain (sc-) and long-chain (Ic-) fructo-oligosaccharides (FOS) were commercially available as Raftilose P95 (Orafti) and Raftiline HP, respectively.

All groups were pretreated with the pepmix and one of the above diets, were sensitized with whey + cholera toxin and challenged with whey.

After challenge with whey, the delta ear swelling of the mice having consumed (c) scFOS/lcFOS diet was about 174 µm, of the mice having consumed (b) the probiotic diet was about 158 µm, and of the mice having consumed (a) the synbiotic diet was about 112 µm. The ear swelling in the synbiotic group (a) was statistically significantly lower than in the corresponding scFOS/lcFOS group (c), and there was a trend of a lower response when compared with probiotic group (b) (p=0.08). These results are indicative for a further improved effect on oral immunotolerance induction:
- when using a lactic acid-producing bacterium, in particular a *Bifidobacterium* strain from the species *B. breve,* and
- particularly when using a lactic-acid producing bacterium in combination with NDOs.

### Example 6: MHC binding domains in the natural, identified peptides of the hydrolysate vs synthetic beta-lactoglobulin peptides

Method: The sequences of the beta-lactoglobulin peptides that were identified in hydrolyzed infant formula (ID_PEP) and the sequences of the synthetic beta-lactoglobulin peptides that have been published in WO2016/148572 (SYN_PEP, Table 9) were tested in silico with the IEDB MHC Class II Binding Prediction Software (http://tools.iedb.org/mhcii/) to determine the number of MHC Class II binding domains in each peptide. In the software the IEDB recommended prediction method and the full HLA reference set were selected. This HLA reference set provides a population coverage of >99%. The software calculates a percentile rank by comparing the binding affinity of the predicted binding domain to a large set of peptides. The higher affinity, the lower the percentile rank. In this experiment, the top 2% binders were selected.

### Results

The number of MHC class II binding domains in the identified peptides is higher than in synthetic peptides (see Table 9). The software predicted at least one binding domain for all identified peptides and in total six different domains. For the synthetic peptides four different domains were predicted. However, these domains were all derived from one sequence, namely SYN_PEP_1. No MHC Class II binding domains were predicted for the other two synthetic peptides.

**Table 9. Number of MHC class II binding domains per betalactoglobulin peptide predicted IEBD**

| Peptide | Sequence | Number of MHC class II binding domains |
|---|---|---|
| ID_PEP_1 | LIVTQTMKGLDIQKVAGTWYSLAMAAS | 1 |
| ID_PEP_2 | LIVTQTMKGLDIQKVAGTWYSLAMAASDISLL | 6 |
| ID_PEP_3 | TMKGLDIQKVAGTWYSLAMAASDISLL | 6 |
| ID_PEP_4 | TMKGLDIQKVAGTWYSLAMAASDISLLDAQ | 6 |
| ID_PEP_5 | DIQKVAGTWYSLAMAASDISLLDAQSAPLRVY | 6 |
| SYN_PEP_1 | QKVAGTWYSLAMAASDIS | 4 |
| SYN_PEP_2 | WYSLAMAASDISLLDAQS | 0 |
| SYN_PEP_3 | AASDISLLDAQSAPLRVY | 0 |

The number of MHC Class II binding domains is higher in the identified peptides than in the synthetic peptides. In other words, more T cell epitopes can be derived from the identified peptides than from the synthetic peptides. Therefore, the likelihood that the identified peptides will be presented to T cells is higher. This presentation to T cells is a requirement for oral immune tolerance induction.

### Example 7: HLA-DR expression on dendritic cells after maturation with a lactic acid producing bacterium

Method: Monocytes (CD14+ cells) were cultured for 7 days with GM-CSF and IL-4 to generate immature dendritic cells (DCs). After 7 days, the immature DCs were washed and incubated with either medium, LPS (100 ng/ml) or *Bifidobacterium breve* M-16V (Morinaga) in a 10:1 bacteria:DC ratio. After 48 hrs, the mature DCs were harvested and stained with APC-Cyanine7 labelled anti-human HLA-DR antibody to determine HLA-DR expression on the surface of the cells. The samples were analyzed on a BD FACS Canto flow cytometer with FACS DIVA software. Expression of HLA-DR is presented in mean fluorescence intensity (MFI).

Results: Maturation with Bifidobacterium breve M-16V (M16v-DCs) increases the expression of HLA-DR to a similar level as LPS (LPS-DCs, see Table 10).

**Table 10. The expression of HLA-DR on different subtypes of dendritic cells**

| DCs | HLA-DR expression MFI (SD) |
|---|---|
| Immature DCs | 11338 (1919,1) |
| LPS-DCs | 24676 (9694,4) |
| M16v-DCs | 21714 (1516) |

| | |
|---|---|
| DCs = dendritic cells, MFI = mean fluorescence intensity, SD = standard deviation | |

*Bifidobacterium breve* M-16V increases the expression of HLA-DR molecules on the surface of the dendritic cells. An increased expression of HLA-DR is indicative for an increased presentation of peptides to T cells.

### Example 8: Infant formula

A powdered infant formula packed with instructions that it should be reconstituted with water of 40 °C with 3 scoops of powder (13.74 g) added to 90 ml water, giving a final volume of 100 ml. The product is suitable from birth up to 6 months.

Per 100 ml the infant formula contains 66 kcal, 1.5 g protein (the hydrolysed whey protein of example 1), 3.3 g fat (vegetable oils, single cell oil and fish oil, comprising 0.4 wt% DHA and 0.35 wt% ARA based on total fatty acids), 7.2 g digestible carbohydrates (mainly lactose), 0.8 g non-digestible oligosaccharides (a 9/1 w/w mix of scGOS and IcFOS), *Bifidobacterium breve* M-16V in an amount of about 3*10⁷ cfu/g, and vitamins, minerals, trace elements and other micronutrients according to international directives for infant formulae.

## Claims

1. A nutritional composition comprising a protein hydrolysate from mammalian milk, preferably milk from a species of the genus *Bos, Bison, Bubalus* or *Capra,* more preferably from genus *Bos*, preferably from cow's milk, for use in
- induction of oral immune tolerance against milk protein; and/or
- prevention or treatment of oral immune intolerance against milk protein; and/or
- reducing the risk of developing oral immune intolerance against milk protein,
in a human subject, wherein the protein hydrolysate comprises at least a peptide having a sequence according to SEQ ID NO: 5 and at least one peptide having a sequence according to SEQ ID NO: 2, SEQ ID NO: 3 or SEQ ID NO: 4,
said composition preferably further comprising at least one strain of a lactic acid-producing bacterium.

2. The nutritional composition for use according to claim 1, wherein the composition comprises less than 6 µg allergenic beta-lactoglobulin, preferably less than 3.5 µg allergenic beta-lactoglobulin, per g of total protein.

3. The nutritional composition for use according to any one of the preceding claims, wherein the composition comprises at least 50 wt%, preferably at least 95 wt%, hydrolysed whey protein based on total protein.

4. The nutritional composition for use according to any one of the preceding claims, comprising less than 10 wt%, preferably less than 6 wt%, of peptides or proteins having a size of 5 kDa or above, based on total protein; and/or wherein at least 1 wt% of peptides or proteins present in the composition has a size of 1 kDa or above, based on total protein, preferably at least 5 wt%, more preferably at least 10 wt%, based on total protein.

5. The nutritional composition for use according to any one of the preceding claims, wherein the composition comprises an amount of allergenic beta-lactoglobulin of above 0.8 µg per g total protein.

6. The nutritional composition for use according to any one of the preceding claims, wherein the milk protein is from a species of the genus *Bos, Bison, Bubalus* or *Capra,* more preferably from genus *Bos*, preferably cow's milk protein, and wherein the human subject is preferably an infant.

7. The nutritional composition for use according to any one of the preceding claims, wherein the composition comprises a strain of lactic acid-producing bacterium belonging to the genus *Bifidobacterium,* preferably to the species *Bifidobacterium breve.*

8. The nutritional composition for use according to any one of the preceding claims, comprising one or more non-digestible oligosaccharide(s) selected from the group consisting of fructooligosaccharide, non-digestible dextrin, galactooligosaccharide, xylooligosaccharide, arabino-oligosaccharide, arabinogalacto-oligosaccharide, gluco-oligosaccharide, glucomanno-oligosaccharide, galactomanno-oligosaccharide, mannan-oligosaccharide, chito-oligosaccharide, uronic acid oligosaccharide, sialyloligosaccharide, and fucooligosaccharide, and mixtures thereof, wherein the non-digestible oligosaccharides preferably comprises at least two non-digestible oligosaccharide(s) selected from the group consisting of fructo-oligosaccharides and galacto-oligosaccharides, preferably a mixture of long-chain fructo-oligosaccharides with short-chain fructo-oligosaccharides or with short-chain galacto-oligosaccharides.

9. The nutritional composition for use according to any one of the preceding claims, comprising long-chain polyunsaturated fatty acids, preferably docosahexaenoic acid (DHA), more preferably at least 0.35 wt.% DHA based on total fatty acids.

10. The nutritional composition for use according to any one of the preceding claims, which is an infant, follow-on formula or young child formula.

11. A nutritional composition comprising:
a. a strain of lactic acid-producing bacterium belonging to the genus *Bifidobacterium;*
b. a milk protein hydrolysate from a species of the genus *Bos, Bison, Bubalus or Capra,* more preferably from genus *Bos*, wherein the milk protein hydrolysate comprises at least a peptide having a sequence according to SEQ ID NO: 5 and at least one peptide having a sequence according to SEQ ID NO: 2, SEQ ID NO: 3 or SEQ ID NO: 4;
c. less than 6 µg allergenic beta-lactoglobulin, preferably less than 3.5 µg beta-lactoglobulin, per g of total protein,
d. less than 10 wt% of peptides or proteins having a size of 5 kDa or above, based on total protein, and
e. at least 50 wt%, preferably at least 95 wt% of hydrolysed whey protein based on total protein,
f. optionally one or more non-digestible oligosaccharide(s) selected from the group consisting of fructooligosaccharide, non-digestible dextrin, galactooligosaccharide, xylooligosaccharide, arabino-oligosaccharide, arabinogalacto-oligosaccharide, gluco-oligosaccharide, glucomanno-oligosaccharide, galactomanno-oligosaccharide, mannan-oligosaccharide, chito-oligosaccharide, uronic acid oligosaccharide, sialyloligosaccharide, and fucooligosaccharide, and mixtures thereof, preferably fructo-oligosaccharides, and
g. optionally long-chain polyunsaturated fatty acids, preferably docosahexaenoic acid (DHA), more preferably at least 0.35 wt.% DHA based on total fatty acids.

12. The nutritional composition according to according to claim 11 wherein the amount of allergenic beta-lactoglobulin is above 0.8 µg per g protein and/or wherein the composition comprises more than 1 wt% of peptides or proteins with a size of 1 kDa or above, based on total protein, more preferably at least 5 wt%, more preferably at least 10 wt%.

13. The nutritional composition according to any one of claims 11 - 12, wherein the strain of lactic acid-producing bacterium belongs to the species *Bifidobacterium breve.*

14. The nutritional composition according to any one of claims 11 - 13, wherein the non-digestible oligosaccharides comprises at least two non-digestible oligosaccharide(s) selected from the group consisting of fructo-oligosaccharides and galacto-oligosaccharides, preferably a mixture of long-chain fructo-oligosaccharides with short-chain fructo-oligosaccharides or with short-chain galacto-oligosaccharides.

15. The nutritional composition according to any one of claims 11 - 14, which is an infant formula, follow-on formula or young child formula.

## Patentansprüche

1. Nährstoffzusammensetzung, umfassend ein Proteinhydrolysat aus Säugetiermilch, vorzugsweise Milch von einer Spezies der Gattung *Bos, Bison, Bubalus* oder *Capra,* noch bevorzugter von der Gattung *Bos*, vorzugsweise aus Kuhmilch, zur Anwendung in
- der Induktion einer oralen Immuntoleranz gegen Milchprotein; und/oder
- der Vorbeugung oder Behandlung von oraler Immunintoleranz gegen Milchprotein; und/oder
- der Verringerung des Risikos, eine orale Immunintoleranz gegen Milchprotein zu entwickeln,
bei einem menschlichen Subjekt, wobei das Proteinhydrolysat mindestens ein Peptid mit einer Sequenz gemäß SEQ ID NO: 5 und mindestens ein Peptid mit einer Sequenz gemäß SEQ ID NO: 2, SEQ ID NO: 3 oder SEQ ID NO: 4 umfasst,
wobei die Zusammensetzung vorzugsweise weiter mindestens einen Stamm eines Milchsäure produzierenden Bakteriums umfasst.

2. Nährstoffzusammensetzung zur Anwendung nach Anspruch 1, wobei die Zusammensetzung weniger als 6 µg allergenes Beta-Lactoglobulin, vorzugsweise weniger als 3,5 µg allergenes Beta-Lactoglobulin, pro Gramm Gesamtprotein umfasst.

3. Nährstoffzusammensetzung zur Anwendung nach einem der voranstehenden Ansprüche, wobei die Zusammensetzung mindestens 50 Gew.-%, vorzugsweise mindestens 95 Gew.-%, hydrolysiertes Molkenprotein, bezogen auf das Gesamtprotein, umfasst.

4. Nährstoffzusammensetzung zur Anwendung nach einem der voranstehenden Ansprüche, umfassend weniger als 10 Gew.-%, vorzugsweise weniger als 6 Gew.-%, an Peptiden oder Proteinen mit einer Größe von 5kDa oder mehr, bezogen auf das Gesamtprotein; und/oder wobei mindestens 1 Gew.-% der in der Zusammensetzung vorhandenen Peptide oder Proteine eine Größe von 1kDa oder mehr, bezogen auf das Gesamtprotein, aufweisen, vorzugsweise mindestens 5 Gew.-%, noch bevorzugter mindestens 10 Gew.-%, bezogen auf das Gesamtprotein.

5. Nährstoffzusammensetzung zur Anwendung nach einem der voranstehenden Ansprüche, wobei die Zusammensetzung eine Menge an allergenem Beta-Lactoglobulin von über 0,8 µg pro g Gesamtprotein umfasst.

6. Nährstoffzusammensetzung zur Anwendung nach einem der voranstehenden Ansprüche, wobei das Milchprotein von einer Spezies der Gattung *Bos, Bison, Bubalus* oder *Capra* stammt, vorzugsweise von der Gattung *Bos*, vorzugsweise Kuhmilchprotein, und wobei das menschliche Subjekt vorzugsweise ein Säugling ist.

7. Nährstoffzusammensetzung zur Anwendung nach einem der voranstehenden Ansprüche, wobei die Zusammensetzung einen Stamm eines Milchsäure produzierenden Bakteriums umfasst, das zu der Gattung *Bifidobacterium,* vorzugsweise zu der Spezies *Bifidobacterium breve,* gehört.

8. Nährstoffzusammensetzung zur Anwendung nach einem der voranstehenden Ansprüche, umfassend ein oder mehrere unverdauliche(s) Oligosaccharid(e), ausgewählt aus der Gruppe bestehend aus Fructo-Oligosaccharid, unverdaulichem Dextrin, Galacto-Oligosaccharid, Xylo-Oligosaccharid, Arabino-Oligosaccharid, Arabinogalacto-Oligosaccharid, Gluco-Oligosaccharid, Glucomanno-Oligosaccharid, Galaktomanno-Oligosaccharid, Mannan-Oligosaccharid, Chito-Oligosaccharid, Uronsäure-Oligosaccharid, Sialyl-Oligosaccharid und Fuco-Oligosaccharid und Mischungen dieser, wobei die unverdaulichen Oligosaccharide vorzugsweise mindestens zwei unverdauliche Oligosaccharide umfassen, die aus der Gruppe ausgewählt sind, die aus Fructo-Oligosacchariden und Galacto-Oligosacchariden besteht, vorzugsweise eine Mischung aus langkettigen Fructo-Oligosacchariden mit kurzkettigen Fructo-Oligosacchariden oder mit kurzkettigen Galacto-Oligosacchariden.

9. Nährstoffzusammensetzung zur Anwendung nach einem der voranstehenden Ansprüche, umfassend langkettige mehrfach ungesättigte Fettsäuren, vorzugsweise Docosahexaensäure (DHA), bevorzugter mindestens 0,35 Gew.-% DHA, bezogen auf die Gesamtfettsäuren.

10. Nährstoffzusammensetzung zur Anwendung nach einem der voranstehenden Ansprüche, die eine Säuglingsanfangsnahrung, Folgenahrung oder Kleinkindnahrung ist.

11. Nährstoffzusammensetzung, umfassend
a. einen Stamm eines Milchsäure produzierenden Bakteriums, das zu der Gattung *Bifidobacterium* gehört;
b. ein Milchproteinhydrolysat aus einer Spezies der Gattung *Bos, Bison, Bubalus* oder *Capra,* vorzugsweise aus der Gattung *Bos*, wobei das Milchproteinhydrolysat mindestens ein Peptid mit einer Sequenz gemäß SEQ ID NO: 5 und mindestens ein Peptid mit einer Sequenz gemäß SEQ ID NO: 2, SEQ ID NO: 3 oder SEQ ID NO: 4 umfasst;
c. weniger als 6 µg allergenes Beta-Lactoglobulin, vorzugsweise weniger als 3,5 µg Beta-Lactoglobulin, pro g des Gesamtproteins,
d. weniger als 10 Gew.-% an Peptiden oder Proteinen mit einer Größe von 5 kDa oder mehr, bezogen auf das Gesamtprotein, und
e. mindestens 50 Gew.-%, vorzugsweise mindestens 95 Gew.-%, hydrolysiertes Molkenprotein, bezogen auf das Gesamtprotein,
f. optional ein oder mehrere unverdauliche(s) Oligosaccharid(e), ausgewählt aus der Gruppe bestehend aus Fructo-Oligosaccharid, unverdaulichem Dextrin, Galacto-Oligosaccharid, Xylo-Oligosaccharid, Arabino-Oligosaccharid, Arabinogalacto-Oligosaccharid, Gluco-Oligosaccharid, Glucomanno-Oligosaccharid, Galaktomanno-Oligosaccharid, Mannan-Oligosaccharid, Chito-Oligosaccharid, Uronsäure-Oligosaccharid, Sialyl-Oligosaccharid und Fuco-Oligosaccharid und Mischungen dieser, vorzugsweise Fructo-Oligosaccharide, und
g. optional langkettige mehrfach ungesättigte Fettsäuren, vorzugsweise Docosahexaensäure (DHA), bevorzugter mindestens 0,35 Gew.-% DHA, bezogen auf die Gesamtfettsäuren.

12. Nährstoffzusammensetzung nach Anspruch 11, wobei die Menge des allergenen Beta-Lactoglobulin über 0,8 µg pro g Protein liegt und/oder wobei die Zusammensetzung mehr als 1 Gew.-% an Peptiden oder Proteinen mit einer Größe von 1 kDa oder mehr, bezogen auf das Gesamtprotein, umfasst, bevorzugter mindestens 5 Gew.-%, noch bevorzugter mindestens 10 Gew.-%.

13. Nährstoffzusammensetzung nach einem der Ansprüche 11-12, wobei der Stamm des milchsäureproduzierenden Bakteriums zu der Spezies *Bifidobacterium breve* gehört.

14. Nährstoffzusammensetzung nach einem der Ansprüche 11-13, wobei die unverdaulichen Oligosaccharide mindestens zwei unverdauliche Oligosaccharide umfassen, die aus der Gruppe bestehend aus Fructo-Oligosacchariden und Galacto-Oligosacchariden ausgewählt sind, vorzugsweise eine Mischung aus langkettigen Fructo-Oligosacchariden mit kurzkettigen Fructo-Oligosacchariden oder mit kurzkettigen Galacto-Oligosacchariden.

15. Nährstoffzusammensetzung nach einem der Ansprüche 11-14, die eine Säuglingsanfangsnahrung, Folgenahrung oder Kleinkindernahrung ist.

## Revendications

1. Composition nutritionnelle comprenant un hydrolysat de protéine provenant de lait de mammifère, de préférence du lait d'une espèce du genre *Bos, Bison, Bubalus* ou *Capra,* plus préférablement du genre *Bos,* de préférence du lait de vache, à utiliser dans
- l'induction d'une tolérance immunitaire orale aux protéines de lait ; et/ou
- la prévention ou le traitement de l'intolérance immunitaire orale aux protéines de lait ; et/ou
- la réduction du risque de développer une intolérance immunitaire orale aux protéines de lait, chez un sujet humain, dans laquelle l'hydrolysat de protéine comprend au moins un peptide présentant une séquence selon SEQ ID NO : 5 et au moins un peptide présentant une séquence selon SEQ ID NO : 2 ; SEQ ID NO : 3 ou SEQ ID NO : 4,
ladite composition comprenant de préférence en outre au moins une souche d'une bactérie produisant de l'acide lactique.

2. Composition nutritionnelle à utiliser selon la revendication 1, dans laquelle la composition comprend moins de 6 µg de bêta-lactoglobuline allergénique, de préférence moins de 3,5 µg de bêta-lactoglobuline allergénique, par g de protéines totales

3. Composition nutritionnelle à utiliser selon l'une quelconque des revendications précédentes, dans laquelle la composition comprend au moins 50 % en poids, de préférence au moins 95 % en poids, de protéine de lactosérum hydrolysée sur la base des protéines totales.

4. Composition nutritionnelle à utiliser selon l'une quelconque des revendications précédentes, comprenant moins de 10 % en poids, de préférence moins de 6 % en poids, de peptides ou de protéines présentant une taille de 5 kDa ou plus, sur la base des protéines totales ; et/ou dans laquelle au moins 1 % en poids de peptides ou de protéines présents dans la composition présentent une taille de 1 kDa ou plus, sur la base des protéines totales, de préférence au moins 5 % en poids, de manière plus préférée au moins 10 % en poids, sur la base des protéines totales

5. Composition nutritionnelle à utiliser selon l'une quelconque des revendications précédentes, dans laquelle la composition comprend une quantité de bêta-lactoglobuline allergénique supérieure à 0,8 µg par g de protéines totales.

6. Composition nutritionnelle à utiliser selon l'une quelconque des revendications précédentes, dans laquelle la protéine de lait provient d'une espèce du genre *Bos, Bison, Bubalus* ou *Capra,* de manière plus préférée du genre *Bos,* de préférence de la protéine de lait de vache, et dans laquelle le sujet humain est de préférence un nourrisson.

7. Composition nutritionnelle à utiliser selon l'une quelconque des revendications précédentes, dans laquelle la composition comprend une souche de bactérie produisant de l'acide lactique appartenant au genre *Bifidobacterium,* de préférence à l'espèce *Bifidobacterium breve.*

8. Composition nutritionnelle à utiliser selon l'une quelconque des revendications précédentes, comprenant un ou plusieurs oligosaccharides non digestibles choisis dans le groupe constitué de fructooligosaccharide, de dextrine non digestible, de galactooligosaccharide, de xylooligosaccharide, d'arabino-oligosaccharide, d'arabinogalacto-oligosaccharide, de gluco-oligosaccharide, de glucomanno-oligosaccharide, de galactomanno-oligosaccharide, de mannane-oligosaccharide, de chito-oligosaccharide, d'oligosaccharide d'acide uronique, de sialyloligosaccharide et de fucooligosaccharide, et de mélanges de ceux-ci, dans laquelle les oligosaccharides non digestibles comprennent de préférence au moins deux oligosaccharides non digestibles choisis dans le groupe constitué de fructo-oligosaccharides et de galacto-oligosaccharides, de préférence un mélange de fructo-oligosaccharides à longue chaîne avec des fructo-oligosaccharides à chaîne courte ou avec des galacto-oligosaccharides à chaîne courte.

9. Composition nutritionnelle à utiliser selon l'une quelconque des revendications précédentes, comprenant des acides gras polyinsaturés à longue chaîne, de préférence de l'acide docosahexaénoïque (DHA), plus préférablement au moins 0,35 % en poids de DHA sur la base des acides gras totaux.

10. Composition nutritionnelle à utiliser selon l'une quelconque des revendications précédentes, qui est une préparation pour nourrisson, une préparation de suite ou préparation pour jeune enfant.

11. Composition nutritionnelle comprenant :
a. une souche de bactérie produisant de l'acide lactique appartenant au genre *Bifidobacterium* ;
b. un hydrolysat de protéines de lait d'une espèce du genre *Bos, Bison, Bubalus* ou *Capra,* plus préférablement du genre *Bos,* dans lequel l'hydrolysat de protéines de lait comprend au moins un peptide présentant une séquence selon SEQ ID NO : 5 et au moins un peptide présentant une séquence selon SEQ ID NO : 2 ; SEQ ID NO : 3 ou SEQ ID NO : 4 ;
c. moins de 6 µg de bêta-lactoglobuline allergénique, de préférence moins de 3,5 µg de bêta-lactoglobuline, par g de protéines totales,
d. moins de 10 % en poids de peptides ou de protéines présentant une taille de 5 kDa ou plus, sur la base des protéines totales, et
e. au moins 50 % en poids, de préférence au moins 95 % en poids de protéines de lactosérum hydrolysées par rapport aux protéines totales,
f. facultativement un ou plusieurs oligosaccharides non digestibles sélectionnés dans le groupe constitué par le fructooligosaccharide, la dextrine non digestible, le galactooligosaccharide, le xylooligosaccharide, l'arabino-oligosaccharide, l'arabinogalacto-oligosaccharide, le gluco-oligosaccharide, le glucomanno-oligosaccharide, le galactomanno-oligosaccharide, le mannan-oligosaccharide, le chito-oligosaccharide, l'oligosaccharide d'acide uronique, le sialyloligosaccharide et le fucooligosaccharide, et des mélanges de ceux-ci, de préférence des fructo-oligosaccharides, et
g. facultativement des acides gras polyinsaturés à longue chaîne, de préférence l'acide docosahexaénoïque (DHA), de manière plus préférée au moins 0,35 % en poids de DHA sur la base des acides gras totaux.

12. Composition nutritionnelle selon la revendication 11,
dans laquelle la quantité de bêta-lactoglobuline allergène est supérieure à 0,8 µg par g de protéine et/ou dans lequel la composition comprend plus de 1 % en poids de peptides ou de protéines d'une taille de 1 kDa ou plus, par rapport aux protéines totales, plus préférentiellement au moins 5 % en poids, plus préférentiellement au moins 10 % en poids.

13. Composition nutritionnelle selon l'une quelconque des revendications 11 à 12, dans laquelle la souche de bactérie produisant de l'acide lactique appartient à l'espèce *Bifidobacterium breve.*

14. Composition nutritionnelle selon l'une quelconque des revendications 11 à 13, dans laquelle les oligosaccharides non digestibles comprennent au moins deux oligosaccharides non digestibles sélectionnés dans le groupe comprenant des fructo-oligosaccharides et des galacto-oligosaccharides, de préférence un mélange de fructo-oligosaccharides à longue chaîne avec des fructo-oligosaccharides à chaîne courte ou avec des galacto-oligosaccharides à chaîne courte.

15. Composition nutritionnelle selon l'une quelconque des revendications 11 à 14, qui est une formule pour nourrisson, une formule de suite ou une formule pour jeune enfant.
